# NEUE EUROPÄISCHE PATENTSCHRIFT

(11) **EP 0 593 896 B2**
(45) Veröffentlichungstag und Bekanntmachung des Hinweises auf die Entscheidung über den Einspruch: **02.03.2005**
(45) Hinweis auf die Patenterteilung: 20.08.1997
(21) Anmeldenummer: 93114669.0
(22) Anmeldetag: 13.09.1993
(51) Int. Cl.: C07D 339/04

(54) **RS-Thioctsäure mit neuartiger Morphologie**
RS-thioctic acid with a new morphology
Acide RS-thioctique avec une morphologie nouvelle

(30) Priorität: 23.10.1992 DE 4235912
(43) Veröffentlichungstag der Anmeldung: 27.04.1994
(73) Patentinhaber: VIATRIS GmbH & Co. KG, 60314 Frankfurt am Main (DE)
(72) Erfinder: Beisswenger, Thomas, Dr., D-61118 Bad Vilbel (DE); Bethge, Horst, D-63457 Hanau (DE); Goede, Joachim, Dr., D-63457 Hanau (DE); Hübner, Frank, Dr., D-64372 Ober-Ramstadt (DE); Huthmacher, Klaus, Dr., D-63571 Gelnhausen (DE); Klenk, Herbert, Dr., D-63457 Hanau (DE); Möller, Roland, D-63403 Hammersbach (DE)

(56) Entgegenhaltungen:
- EP-A- 0 543 088
- CHEMICAL ABSTRACTS, vol. 76, no. 70, 1972, Columbus, Ohio, US; abstract no. 51202u, R.STROUD ET AL. 'SINGLE-CRYSTAL STRUCTURE DETERMINATION OF DL-6-THIOCTIC ACID' Seite 367 ; & ACTA CRYSTALLOGR. SECT. B Bd. 28, Nr. 1 , 1972 , ENGL. Seiten 304 - 307
- Acker and Wayne;J Am. Chem. Soc. 76 (1954), S. 6483-6487
- Walton et al., J. Am. Chem. Soc. 77 (1955), S. 5144-5149
- Segre et al., J. Am. Chem. Soc. 79 (1957), S. 3503-3505
- Brookes and Golding, J. Chem. Soc. Perkin Trans. 1 (1988), S. 9-12
- CRC Handbook of Chemistry and Physics, 76th edition, CRC Press, 1995-1996, Seiten 6-165,-166, -168, -171, -172

## Beschreibung

RS-Thioctsäure (DL-alpha-Liponsäure) wird in pharmazeutischen Formulierungen sowohl in Infusionslosungen bzw. auch als feste galenische Formulierung zur oralen Anwendung eingesetzt.

Wenn im folgenden von Thioctsäure die Rede ist, sind immer sowohl die enantiomerenreinen Verbindungen (R- oder S-Thioctsäure) als auch das racemische Gemisch (R,S-Thioctsäure) und Mischungen mit beliebigen Gehalten an Enantiomeren zu verstehen.

Verwendet wird hierfür auf synthetischem Wege gewonnene DL-Thioctsäure, die über die Vorstufe der Dihydroliponsaüre (6,8-Dimercaptooctansäure) durch Oxidation gewonnen wird. Das synthetische Verfahren kann hier auch so durchgeführt werden, daß aus enantiomerenreiner R- oder S-Dihydroliponsäure enantiomerenreine R- oder S-Thioctsäure erzeugt und vorteilhaft eingesetzt werden kann (D- oder L-alpha-Liponsäure) DE 41 37 773.7, EP 0427247. Enantiomerenreine R-Thioctsäure und S-Thioctsäure ist auch nach dem in der DE-OS 36 29 116 beschriebenen Verfahren zugänglich.

Die bisher erhältliche Ware lag in einer für die pharmazeutische Verarbeitung nicht sehr günstigen Form vor: sie war nicht rieselfähig, was zu Ungleichmäßigkeiten bei der Füllung führte und einen erhöhten Einsatz an Hilfsstoffen, oder eine intensive Granulation erforderte, um mit diesem Wirkstoff pharmazeutische Tabletten herzustellen.
Darüberhinaus neigt sie zum Stauben, was sich vom Standpunkt des Arbeitsschutzes als nachteilig erweist.

Aufgabe der vorliegenden Erfindung war die Entwicklung eines Verfahrens zur Herstellung von reiner, pharmazeutisch einsetzbarer Thioctsäure in einer für die galenische Verarbeitbarkeit günstigen Form.

Literaturbekannt sind hierfür Verfahren (Chem. Ber. 1959, 1177), bei denen die Dimercaptooctansäure destilliert und nach Oxidation zur Thioctsäure erneut durch Destillation gereinigt wird, um dann schließlich bei -70 °C aus Essigester kristallin erhalten zu werden. Eine weitere Methode (J.Am.Chem.Soc. 77(1955), 416) verwendet das nach der Oxidation von Dimercaptooctansäure durch Einengen der organischen Lösungsmittel erhältliche viskose Öl. Dieses wird mit Skellysolve B mehrfach extrahiert wobei ein wechselnder Anteil eines "polymeren" Materials zurückbleibt.

Die vereinigten Extrakte werden angeimpft und bei Raumtemperatur oder während einiger Stunden im Kühlschrank kristallisiert. Die Umkristallisation aus Skellysolve B ergibt schließlich analysenreines Produkt mit einem Schmelzpunkt von 61 °C - 62 °C.

Weitere Vorschriften (J.Am.Chem.Soc. 77(1955) 5144-5149; J.Chem. Soc. 76(1954), 6483-6487; J.Chem. Soc. 79(1957), 3503-3505; J.Chem.Soc. Perkin Trans. 1(1988), 9-12 emptehlen zur Extraktion und Kristallisation Pentan, Gemische aus Pentan und Hexan, z.B. Petroletter, und Cyclohexan wobei auch enantiomerenreine (+)-alpha-Liponsäure auf analogem Wege erhalten wurde (J. Chem. Soc. Perkin Trans. 1(1990), 1615).
Derartig erzeugte Kristallisate weisen die von Reed et al., J.Am.Chem.Soc., 7(1953)1267 für (+)-alpha-Liponsaure und die von ElLi Lilly für DL-alpha-Liponsäure publizierten Röntgen-Diffraktogramme auf. [Transmissions-Röntgen Diffraktometer Aufnahmen mit Cu K_{α1}-Strahlung (2 Theta)]. Nähere Ausführungen zur Rögendiffraktometrie finden sich in U.S. Pharmacopeia XXII, 1989, S. 1621-1623, H.P. Klug & L.E. Alexander, X-Ray Diffraction Procedures for Poly-crystalline and Amorphuns Materials ; Ind Edition, S. 285-288, John Wiley, Interscience Publication, 1974, H. W. King & L. F. Vassamillet, Advantages in X-Ray Analysis, Vol. 5, Plenum Press, S. 78-85, 1962, und L. Vassamillet & H.W. King, Advances in X-Ray Analysis, Vol.6, Plenum Press, S, 142-147, 1963.

Die erfindungsgemäße Aufgabe wurde nun so gelost, daß das durch Oxidation des 6,8-Dimercaptooctansäure-Natriumsalzes in wäßriger Losung entstehende Natriumsalz der alpha-Liponsäure durch Zusatz von Mineralsäuren in die freie alpha-Liponsäure überführt wird und dabei direkt in ein organisches Lösungsmittel mit einer Dielektrizitätskonstante epsilon von 2,5 bis 5,5 bei Raumtemperatur extrahiert wird. Die entstehende organische alpha-Liponsäurelösung wird dann nach vollständiger Phasentrennung langsam auf +10 °C bis -20 °C abgekühlt, wobei sowohl für racemische als auch für enantiomerenreine alpha-Liponsäure ein neuartiges Kristallisat entsteht, das ein Röntgen-Diffraktogramm mit einer neuartigen Linienintensitätsverteilung sowie eine für die Verarbeitung geeignete Korngrößenverteilung und Partikelbeschaffenheit aufweist. Darüberhinaus ist bei der erfindungsgemäßen Ware die Auflösegeschwindigkeit in wässrigem Phosphat-Puffer (pH 6,8) im Vergleich zur handelsüblichen Ware erhöht und es treten weniger Klumpen auf.

Die Verwendung der erfindungsgemäßen Lösungsmittel mit einer Dielektrizitätskonstante von 2,5 bis 5,5 haben darüberhinaus gegenüber den literaturbeschriebenen den Vorteil, daß sie bei der Kristallisation ein sehr reines Kristallisat liefern, während beispielsweise eine Fällung aus Cyclohexan auch zum Einschluß von Nebenverbindungen führt. Als Lösungsmittel können beispielsweise dienen: aliphatische Kohlenwasserstoffe mit einer Kettenlänge von C₅ - C₇, cycloaliphatische Kohlenwasserstoffe wie Cyclopentan, Cyclohexan, Methylcyclohexan Carbonsäureester von aliphatischen Carbonsäuren wie Essigsäure und Propionsäure mit aliphatischen Alkoholen wie Methanol, Ethanol oder Propanol sowie Ether aus aliphatischen Alkoholen mit einer Kettenlänge von 1-4 Kohlenstoffatomen.

Die Ether können auch ringgeschlossen sein, wie beispielsweise Tetrahydrofuran oder Dioxan. Auch Mischungen der vorgenannten Lösungsmittel können eingesetzt werden.

Bei der Synthese von Thioctsäure anfallende Nebenprodukte sind beispielsweise 6-Mercapto-8-chloroctansäure oder 6-Chlor-8-mercaptooctansäure, die nach Oxidation des Stoffgemisches mit 6,8-Dimercaptooctansäure polymere Disulfide bilden. Die Disulfide müssen, um eine pharmazeutische Anwendung zu erlauben, mit der Mutterlauge abgetrennt werden.

Die der Erfindung zugrundeliegende Aufgabe, während der Synthese anfallende Nebenprodukte ohne Destillation der 6,8-Dimercaptooctansäure oder der Thioctsäure selbst zu entfernen, kann so einfach und ohne die sonst zu erwartende Zersetzung der Octansäuren erreicht werden.

Die erfindungsgemäße Ware löst sich darüberhinaus schneller als Ware gemäß des Standes der Technik und bildet keine Agglomerate.

Die erfindungsgemäße Ware besitzt in wäßriger Lösung (1 g Thioctsäure in 20 1 N NaOH) eine Extinktion von < 0,300 (430 nm), Schichtdicke 1 cm.

### Beispiel 1

| | |
|---|---|
| Einsatzmengen | 50,0 g R(+)-α-Liponsäure 500 ml Gemisch aus Cyclohexan/Essigester 2:1 |

### Durchführung:

50 g R(+)-α-Liponsäure wurden in 500 ml Lösungsmittelgemisch bei Raumtemperatur eingetragen.
Die Temperatur sank auf 10 °C und nach wenigen Minuten erhielt man eine klare Lösung. Geringe Mengen Polymeranteile blieben ungelöst zurück.
Die Lösung wurde abdekantiert und langsam unter Rühren mit Eiswasser abgekühlt. Anschließend wurde mit Eis/Kochsalz-Gemisch langsam weitergekühlt.
Bei -6 °C begann der Kristallisationsprozeß. Nachdem die Hauptmenge auskristallisiert war, wurde weiter bis auf ca -15 °C abgekühlt und 2 Stunden bei dieser Temperatur nachgerührt.
Anschließend wurde abgesaugt und im Vakuum bei Raumtemperatur getrocknet.

| | |
|---|---|
| Ergebnis | 15,6 g R(+)-α-Liponsäure |
| Ausbeute | 31 % |

Die Mutterlauge kann für weitere Kristallisationen eingesetzt werden.

### Beispiel 2

### Umkristallisation von RS-α-Liponsäure aus Diisopropylether

| | |
|---|---|
| Einsatzmengen | 50,0 g RS-α-Liponsäure 500 ml Diisopropylether |

### Durchführung:

50 g RS-α-Liponsäure wurden in 500 ml Diisopropylether bei Raumtemperatur eingetragen. Mit Warmwasser wurde langsam erwärmt. Bei 33 °C erhielt man eine klare Lösung. Geringe Mengen Polymeranteile blieben ungelöst zurück.
Die Lösung wurde bei 33 °C abfiltriert und langsam unter Rühren mit Eiswasser abgekühlt.

Bei 15-16 °C begann der Kristallisationsprozeß. Nachdem die Hauptmenge auskristallisiert war, wurde mit Eis/Kochsalz-Gemisch langsam weiter bis auf ca. -15 °C abgekühlt und 2 Stunden bei dieser Temperatur nachgerührt.
Anschließend wurde abgesaugt und im Vakuum bei Raumtemperatur getrocknet.

| | |
|---|---|
| Ergebnis | 38,2 g RS-α-Liponsäure |
| Ausbeute | 76 % |

### Beispiel 3

### Umkristallisation von RS-α-Liponsäure aus Pentan/Essigester 2:1

| | |
|---|---|
| Einsatzmengen: | 50,0 g R,S-α-Liponsäure 500 ml Gemisch aus Pentan/Essigester 2:1 |

### Durchführung:

50 g RS-α-Liponsäure wurden in 500 ml Lösungsmittelgemisch bei Raumtemperatur eingetragen. Mit Warmwasser wurde langsam erwärmt.

Bei 35 °C erhielt man eine klare Lösung. Geringe Mengen Polymeranteile blieben ungelöst zurück.
Die Lösung wurde bei 35 °C abfiltriert und langsam unter Rühren mit Eiswasser abgekühlt.
Bei 16-17 °C begann der Kristallisationsprozeß. Nachdem die Hauptmenge auskristallisiert war, wurde mit Eis/Kochsalz-Gemisch langsam weiter bis auf ca. -15 °C abgekühlt und 2 Stunden bei dieser Temperatur nachgerührt.
Anschließend wurde abgesaugt und im Vakuum bei Raumtemperatur getrocknet.

| | |
|---|---|
| Ergebnis: | 40,4 g RS-α-Liponsäure |
| Ausbeute: | 81 % |

### Beispiel 4

### Umkristallisation von RS-α-Liponsäure aus Cyclohexan/Essigester 5:1

| | |
|---|---|
| Einsatzmengen: | 50,0 g RS-α-Liponsäure 500 ml Gemisch aus Cyclohexan/Essigester 5:1 |

### Durchführung:

50 g RS-α-Liponsäure wurden in 500 ml Lösungsmittelgemisch bei Raumtemperatur eingetragen. Mit Warmwasser wurde langsam erwärmt.
Bei 41 °C erhielt man eine klare Lösung. Geringe Mengen Polymeranteile blieben ungelöst zurück.
Die Lösung wurde bei 40 °C abfiltriert und langsam unter Rühren abgekühlt.
Bei 26-27 °C begann der Kristallisationsprozeß.
Nachdem die Hauptmenge auskristallisiert war, wurde mit Eiswasser langsam weiter bis auf ca. 5 °C abgekühlt und 2 Stunden bei dieser Temperatur nachgerührt.
Anschließend wurde abgesaugt und im Vakuum bei Raumtemperatur getrocknet.

| | |
|---|---|
| Ergebnis: | 44,4 g RS-α-Liponsäure |
| Ausbeute: | 89 % |

Die erfindungsgemäß hergestellten Thioctsäuren wurden einer Röntgenbeugungsanalyse mit Cu K-alpha-Strahlung unterzogen.
Figur 1 zeigt eine RS-Thioctsäure gemäß dem Stand der Technik, Figur 2 zeigt die Analyse der erfindungsgemäßen RS-Thioctsäure.
Figur 3 zeigt eine R-Thioctsäure gemäß dem Stand der Technik, Figur 4 zeigt eine Analyse der erfindungsgemäßen R-Thioctsäure.

### Figurenbeschreibung

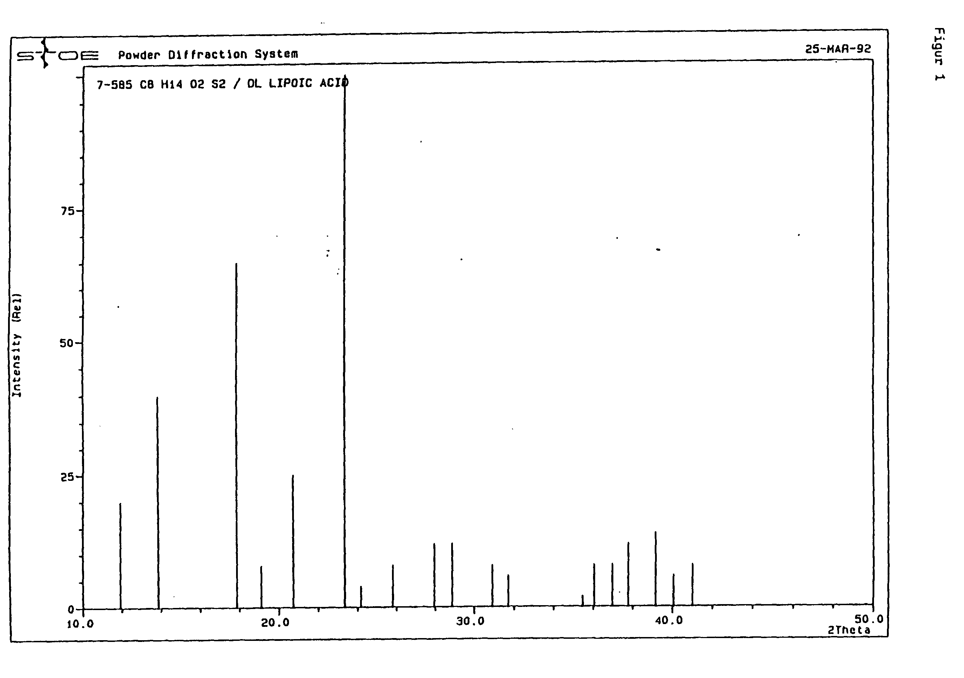

Figur 1 stellt das Pulverdiagramm der Rohware dar. (racemisches Gemisch)

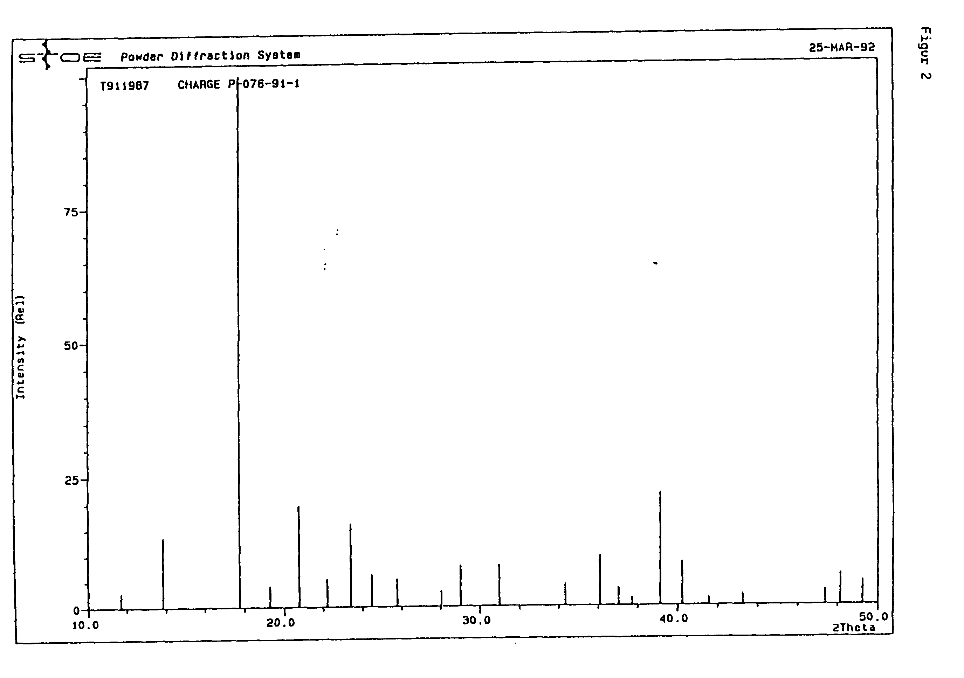

Figur 2 stellt das Pulverdiagramm der erfindungsgemäßen Ware dar. (racemisches Gemisch)

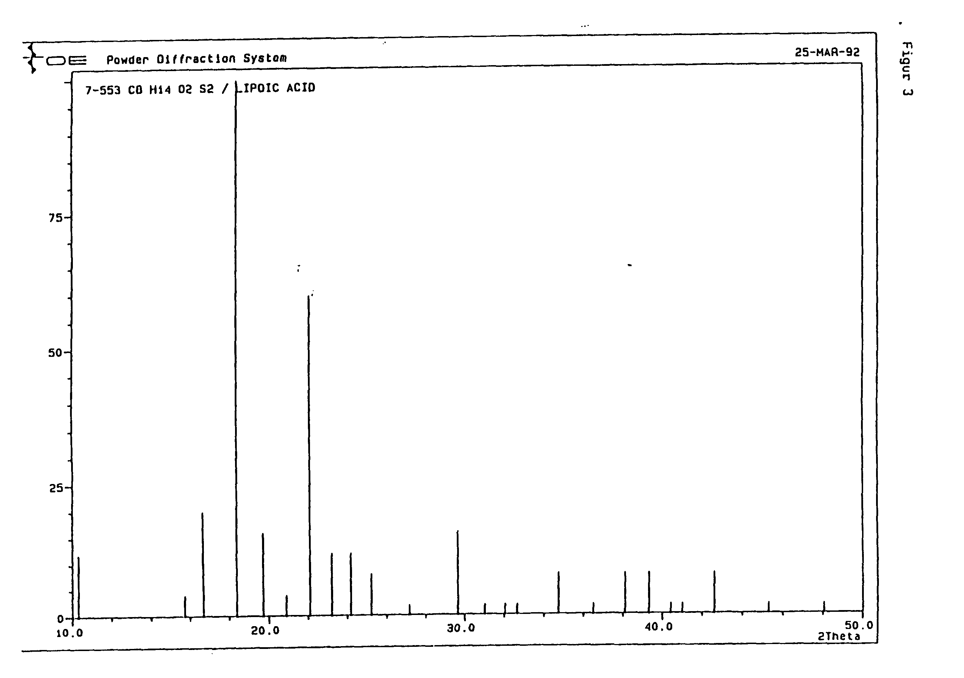

Figur 3 stellt das Pulverdiagramm einer handelsüblichen, enantiomerenreinen R-α-Liponsäure dar

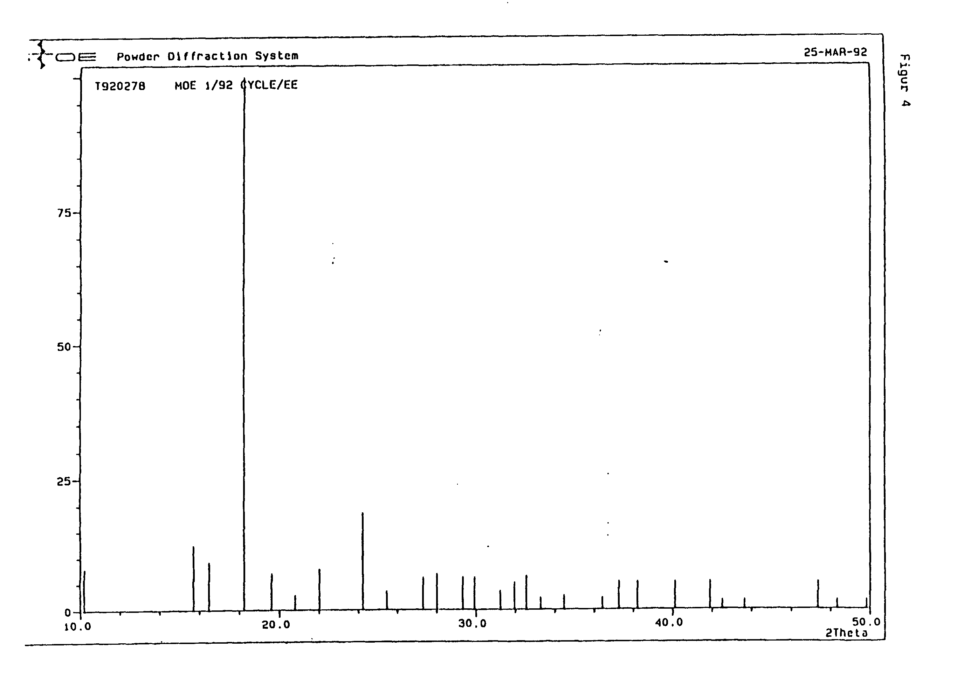

Figur 4 stellt das Pulverdiagramm einer erfindungsgemäßen, enantiomerenreinen R-α-Liponsäure dar.

## Patentansprüche

1. Verfahren zur Herstellung kristalliner Thioctsäure, **dadurch gekennzeichnet, daß** man ein Teil Thioctsäure bei 10 °C bis 60 °C in 5-20 Teilen Lösungsmittel oder Lösungsmittelgemisch löst und innerhalb von 2-10 Stunden auf 0 °C bis -20 °C abkühlt, wobei das Lösungsmittel oder das Lösungsmittelgemisch eine Dielektrizitätskonstante epsilon zwischen 2,5 und 5,5 aufweist.

2. Verfahren nach Anspruch 2, **dadurch gekennzeichnet, daß** als Lösungsmittelgemisch ein Gemisch aus Pentan, Cyclohexan und Essigsäureethylester oder Ethern eingesetzt wird.

3. Thioctsäure, erhältlich nach einem oder mehreren der vorhergegangenen Verfahrensansprüche.

4. Verwendung von Thioctsäure nach Anspruch 3 zur Herstellung von Arzneimitteln.

## Claims

1. A method for preparing crystalline thioctic acid, **characterised in that** one part thioctic acid is dissolved in 5 to 20 parts solvent or solvent mixture at 10°C to 60°C and is cooled to 0°C to -20°C over a period of 2 to 10 hours, the solvent or the solvent mixture having a dielectric constant epsilon of between 2.5 and 5.5.

2. A method according to claim 2, **characterised in that** a mixture of pentane, cyclohexane and ethyl acetate or ethers is used as the solvent mixture.

3. Thioctic acid, obtainable by one ore more of the preceding method claims.

4. The use of thioctic acid according to claim 3 for the production of medicines.

## Revendications

1. Procédé de production d'acide thioctique cristallin, **caractérisé en ce qu'**on dissout une partie de l'acide thioctique à 10°C à 60°C dans 5-20 parties de solvant ou de mélange de solvants, et en l'espace de 2 à 10 heures on refroidit à 0°C à -20°C, procédé dans lequel le solvant ou le mélange de solvants possède une constante diélectrique epsilon comprise entre 2,5 et 5,5.

2. Procédé selon la revendication 2, **caractérisé en ce qu'**on met en oeuvre, comme mélange de solvants, un mélange à base de pentane, de cyclohexane et d'acétate d'éthyle ou d'éthers.

3. Acide thioctique que l'on peut obtenir selon l'une ou plusieurs des revendications de procédé précédents.

4. Utilisation de l'acide thioctique selon la revendication 3 en vue de la production de médicaments.
